# EUROPEAN PATENT APPLICATION

(11) **EP 3 073 291 A1**
(43) Date of publication of application: **28.09.2016**
(21) Application number: 14864485.9
(22) Date of filing: 08.10.2014
(51) Int. Cl.: G01T 1/24, G01T 1/161

(54) **RADIATION DETECTION ELEMENT, RADIATION DETECTOR PROVIDED WITH SAME, NUCLEAR MEDICINE DIAGNOSIS DEVICE AND METHOD FOR PRODUCING RADIATION DETECTION ELEMENT**

(30) Priority: 21.11.2013 JP 2013241044
(71) Applicant: Hitachi, Ltd., Chiyoda-ku, Tokyo 100-8280 (JP)
(72) Inventor: TAKAHASHI Isao, Tokyo 100-8280 (JP); KOMINAMI Shinya, Tokyo 100-8280 (JP); SEINO Tomoyuki, Tokyo 100-8280 (JP)
(74) Representative: MERH-IP Matias Erny Reichl Hoffmann Patentanwälte PartG mbB
(86) International application number: PCT/JP2014/077017
(87) International publication number: WO 2015/076033

(57) **Abstract**

This radiation detection element (1) is provided with: a crystal part (10) that is formed from a mixed ion-electron conductor, which is a compound wherein thallium ions are bonded with bromine ions; a metal electrode (13a) that is formed on one surface of the crystal part (10); and a metal electrode (13b) that is formed on a surface which is on the reverse side of the surface where the metal electrode (13a) is formed. The crystal part (10) has metal-containing parts (12) in the surfaces where the metal electrodes (13a, 13b) are respectively formed, said metal-containing parts (12) containing thallium that is obtained by selectively removing elemental bromine and neutralizing thallium ions. Consequently, there are provided: a practical radiation detection element which is suppressed in the occurrence of problems caused by polarization; and a method for producing this radiation detection element.

## Description

### Technical Field

The present invention relates to a radiation detection element, a radiation detector provided with the same, a nuclear medicine diagnosis device and a method for producing a radiation detection element.

### Background Art

As a radiation detector in which a thallium halide such as TIBr is used, there is, for example, the detector described in Patent Document 1.

This Patent Document 1 describes a radiation detection element using a thallium halide crystal, specifically, a thallium halide radiation detection element wherein thallium metal electrodes are provided on two surfaces opposite to each other.

### Prior Art Document

### Patent Document

Patent Document 1: Japanese Patent No. 5083964

### Summary of the Invention

### Problems to be Solved by the Invention

As a blank material for a radiation detector that detects radiation and outputs a signal, there are used a variety of materials such as scintillator and gas. Among the materials are semiconductors, which have been drawing attention in view of their merits that they are easy to finely process, they enable accurate determination of the incidence position of radiation, or they enable accurate determination of the energy of the radiation detected.

In the case of using a semiconductor as a radiation detection element, in general, metal electrodes are provided on opposite surfaces of a crystal, and a voltage is applied thereon. When electron-hole pairs are generated in the semiconductor by radiation, the electrons and holes are drawn by an electric field to be collected at the electrodes, whereby signals are outputted. The signals are processed, whereby information such as the kind of the radiation and the number of the incident radiations can be obtained.

In order to enhance the sensitivity of the radiation detector to radiation, it is effective to use a radiation detection element which is high in density and atomic number. In view of this, radiation detection elements formed from heavy metal-containing semiconductors such as thallium bromide and mercury iodide have been under development. These semiconductors, unlike silicon and the like, are characterized by their ionic properties strongly observed in their crystals and are, hence, called mixed ionic-electronic conductors.

In a radiation detection element using such a mixed ionic-electronic conductor, not only the electron-hole pairs but also ions are drawn by an electric field, to move into the vicinities of electrodes. Since these ions are collected faster than they are neutralized, the ions form space charges near the electrodes, resulting in that the electric field due to the applied voltage is canceled. This phenomenon is called polarization. This polarization causes an undesirable phenomenon in which after a while from the applying a voltage on the radiation detection element, it becomes impossible to normally output a radiation detection signal.

In order to avoid such polarization, in the radiation detection element described in Patent Document 1, thallium metal electrodes are provided on opposite two surfaces of the radiation detection element in which a thallium halide crystal is used. According to this method, after thallium ions and halogen ions in the thallium halide crystal are moved into the vicinities of electrodes made of thallium metal, the thallium ions are changed into thallium metal, whereas the halogen ions are changed into thallium halide. Thus, the ions are turned into non-ions, in other words, the ions are neutralized. By this method, therefore, a radiation detection element free of occurrence of polarization can be provided.

In Patent Document 1, however, thallium metal electrode films are formed on the thallium halide crystal by vapor deposition. However, heavy metals are often toxic, with thallium listed first, and are therefore not easy to handle. In addition, since it is necessary to vaporize a heavy metal for performing vapor deposition of the heavy metal, the heavy metal must be handled very carefully, so that various measures must be taken therefor. Accordingly, there is a need for further improvement of this method before putting the method into practical use.

The present invention has been made in consideration of the above-mentioned problems. Accordingly, it is an object of the present invention to provide a practical radiation detection element in which polarization-induced problems are restrained from being generated, a radiation detector and a nuclear medicine diagnosis device which are provided with the radiation detection element, and a method for producing the radiation detection element.

### Means for Solving the Problems

In order to solve the above problems, for example, the configurations set forth in the claims are adopted.

The present invention includes a plurality of means for solving the above problems. One example of the solving means is a radiation detection element including: a crystal part formed from a mixed ionic-electronic conductor that is a compound having a heavy metal ion and an anion bonded to each other; a first metal electrode formed on one surface of the crystal part; and a second metal electrode formed on a surface of the crystal part, this surface being opposite to the surface where the first metal electrode is formed, wherein the crystal part has a metal-containing part on each of the surfaces where the first metal electrode and the second metal electrode are formed, the metal-containing part containing a heavy metal obtained by selectively removing an element of the anion and neutralizing the heavy metal ions.

### Advantageous Effect

According to the present invention, it is possible to provide a practical radiation detection element in which polarization-induced problems are restrained from being generated and a method of producing the radiation detection element, and also to provide a radiation detector and a nuclear medicine diagnosis device which are provided with the radiation detection element.

### Brief Description of the Drawings

FIG. 1 is a perspective view showing the configuration of a radiation detection element according to an embodiment of the present invention.
FIG. 2 is a side view showing the configuration of the radiation detection element according to the embodiment of the present invention.
FIG. 3 is a schematic view of circuit configuration of a radiation detector for performing radiation counting by use of the radiation detection element according to the embodiment of the present invention.
FIG. 4 is a block diagram of an example of a nuclear medicine diagnosis device in which the radiation detector according to the embodiment of the present invention is used.
FIG. 5 is a block diagram of another example of a nuclear medicine diagnosis device in which the radiation detector according to the embodiment of the present invention is used.

### Modes for Carrying Out the Invention

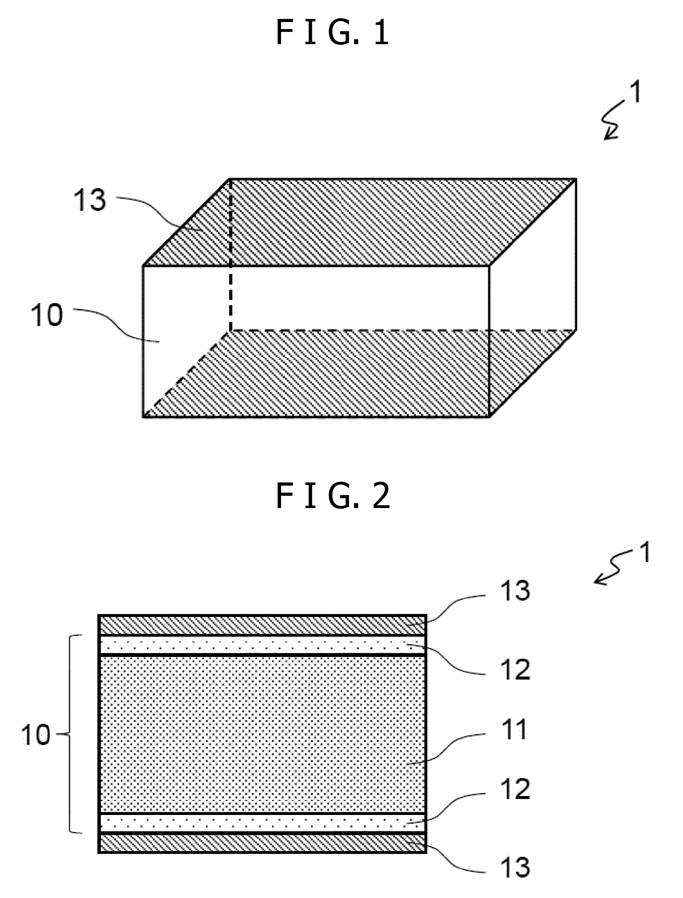

A radiation detection element, a radiation detector provided with the same and a method for producing a radiation detection element according to the present invention will be described, referring to FIGS. 1 to 3.

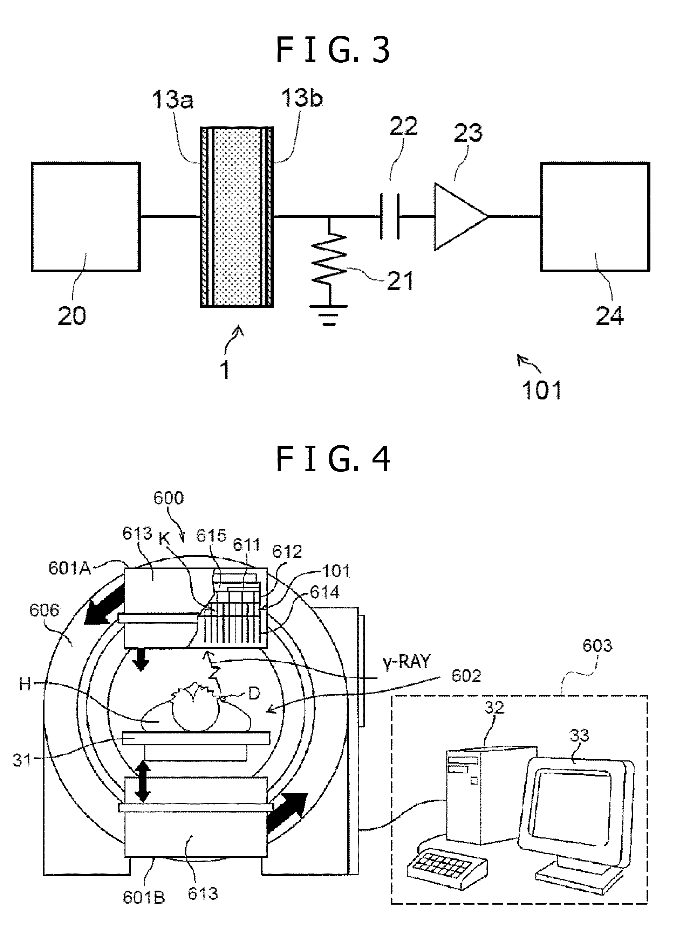

FIG. 1 is a perspective view showing the configuration of a radiation detection element according to an embodiment of the present invention. FIG. 2 is a side view showing the configuration of the radiation detection element according to the embodiment of the present invention. FIG. 3 is a schematic view of circuit configuration of a radiation detector for performing radiation counting by use of the radiation detection element according to the embodiment of the present invention.

### Configuration of Radiation Detection Element

As shown in FIGS. 1 and 2, a radiation detection element 1 includes a crystal part 10 and metal electrodes 13.

The crystal part 10 includes: a single crystal part 11 composed of a single crystal of a mixed ionic-electronic conductor consisting of a compound having a heavy metal ion and an anion bonded to each other; and metal-containing parts 12.

Examples of the specific material of the crystal part 10 include thallium bromide, mercury iodide, lead iodide, bismuth iodide, and indium iodide; in this example, the material is a single crystal of thallium bromide. The size of the crystal part 10 is, for example, 5 mm in width, 4 mm in depth, and 1.5 mm in thickness.

The metal-containing parts 12 are formed on the sides of the surfaces where the metal electrodes 13 are formed, of the crystal part 10. The composition of the metal-containing parts 12 is basically the same as that of the single crystal part 11, wherein thallium obtained by selectively removing the bromide ion as the anion from a thallium bromide crystal and neutralizing the thallium ions is contained as metal.

The metal electrodes 13 are electrodes for applying a voltage on the crystal part 10, and are provided on one surface where the metal-containing part 12 is formed and on a surface opposite to the one surface, of the crystal part 10 of the radiation detection element 1.

The metal electrodes 13 are preferably formed from a metal having a low reactivity, such as, for example, platinum, gold, palladium, and chromium; in this example, the metal electrodes 13 are platinum electrodes. The thickness of the metal electrodes 13 is, for example, 50 nm.

### Circuit Configuration

In the next place, a radiation detector using the radiation detection element 1 according to this embodiment will be described below, referring to FIG. 3.

As illustrated in FIG. 3, a radiation detector 101 has a high voltage power source generation device 20 connected to a metal electrode 13a on one side of the radiation detection element 1. The high voltage power source generation device 20 is a device for applying a voltage of, for example, +1,000 V on the metal electrode 13a of the radiation detection element 1.

A metal electrode 13b formed on a surface opposite to the metal electrode 13a of the radiation detection element 1 is earthed through a resistor 21, such that a potential difference of 1,000 V is generated between the opposite electrodes of the radiation detection element 1.

When radiation is detected by the radiation detection element 1 during when the voltage is applied, an electric charge generated by the energy of the radiation is moved by the potential difference, whereby an induced charge is induced at the metal electrode 13b. This charge is inputted to a charge-sensitive preamplifier 23 via a coupling capacitor 22 connected to the metal electrode 13b. The charge-sensitive preamplifier 23 converts and amplifies a radiation detection signal, which is a minute electric charge, into a voltage. The resulting radiation detection signal is inputted to a signal processor 24 provided at a later stage.

In the signal processor 24, the inputted radiation detection signal is subjected further to such processing as waveform shaping, peak hold, and analog-digital conversion by processing circuits, and the processed signal is processed into information of energy spectrum and the like by a data processor. The information of energy spectrum and the like after the processing are outputted to a display or the like, to be displayed.

### Detection of Radiation and Polarization-Inhibitive Effect

As aforementioned, the radiation detection element 101 of this embodiment includes the radiation detection element 1, and the signal processor 24 that processes the radiation detection signal outputted from either one of the metal electrodes 13a and 13b. Of these components, the radiation detection element 1 includes the crystal part 10 formed from the mixed ionic-electronic conductor that is a compound having a heavy metal ion (thallium ion) and an anion (bromine ion) bonded to each other, the metal electrode 13a formed on one surface of the crystal part 10, and the metal electrode 13b formed on a surface opposite to the surface where the metal electrode 13a is formed, wherein the crystal part 10 has the metal-containing part 12 on each of the surfaces where the metal electrode 13a and the metal electrode 13b are formed, the metal-containing part 12 containing thallium obtained by selectively removing bromine and neutralizing the thallium ions.

Therefore, when a voltage of +1,000 V is applied on the radiation detection element 1 by the high-voltage power source generation device 20 in order to detect radiation by the radiation detector 101, an electric field generated inside the crystal part 10 causes the bromine ions and the thallium ions in the thallium bromide crystal to move. The bromine ions as anions are moved toward the side of the metal electrode 13a, whereas the thallium ions as cations are moved toward the side of the metal electrode 13b.

When the voltage is applied continuedly, the thus moved ions would accumulate in the vicinity of the metal electrode 13a or the metal electrode 13b, to bring about polarization, whereby the electric field in the crystal would be weakened, and the performance of the radiation detector would be deteriorated.

In the radiation detection element 1 in this embodiment, however, the metal-containing parts 12 containing thallium metal are provided beneath the metal electrodes 13a and 13b. Therefore, on the side of the metal electrode 13a, the bromine ions having moved there combine with thallium metal contained in the metal-containing part 12, to be thallium bromide while releasing electrons, and, accordingly, accumulation of the bromine ions on the side of the metal electrode 13a is restrained. On the side of the metal electrode 13b, besides, the thallium ions having moved there combine with electrons to be thallium metal, and, accordingly, accumulation of the thallium ions on the side of the metal electrode 13b is restrained. Owing to these effects, generation of polarization is restrained, and deterioration of the performance of the radiation detector is restrained.

When this process continues, thallium metal contained in the metal-containing part 12 provided beneath the metal electrode 13a turns into thallium bromide, whereby the content of thallium metal would be gradually decreased. Besides, the content of thallium metal in the metal-containing part 12 provided beneath the metal electrode 13b would be increased.

However, the reactions through which thallium bromide and thallium metal are produced are reversible reactions. In view of this, the polarity of the voltage to be applied on the crystal part 10 by the high-voltage power source generation device 20 is changed, and a voltage of -1,000 V is applied on the crystal part 10 for the same period of time.

This results in that on the side of the metal electrode 13b, the bromine ions having moved there combine with thallium metal contained in the metal-containing part 12, to be thallium bromide while releasing electrons, whereby the content of thallium metal is decreased. Similarly, on the side of the metal electrode 13a, the thallium ions having moved there combine with electrons to form thallium metal, whereby the content of thallium metal is increased. By this process, the composition of the metal-containing parts 12 of the crystal part 10 can be returned into the same state as in an initial state before the applying the voltage. Accordingly, the radiation detector in this embodiment can be used permanently.

In addition, also when the voltage of -1,000 V is applied on the crystal part 10, an electric field is generated in the crystal of the crystal part 10. When radiation is detected by the radiation detection element 1, therefore, a radiation signal is inputted to the charge-sensitive preamplifier 23, so that radiation counting can be performed.

Note that since the polarity of the radiation signal inputted to the charge-sensitive preamplifier 23 in the case where the output of the high voltage power source generation device 20 is +1,000V and that in the case where the output is -1,000 V are opposite to each other, the process of the charge-sensitive preamplifier 23 is changed over according to the polarity of the voltage outputted from the high voltage power source generation device.

Besides, while the case of using thallium bromide for the crystal part 10 has been described as above, the material constituting the crystal part 10 may be any mixed ionic-electronic conductor of mercury iodide, lead iodide, bismuth iodide, and indium iodide.

In addition, while the case of using platinum for the metal electrode 13a and the metal electrode 13b has been described above, the material for the electrodes may be any metal of gold, palladium, and chromium, and, further, may be an alloy obtained by mixing at least one metal of gold, platinum, palladium, and chromium.

### Production of Radiation Detection Element

In the next place, a method for producing the radiation detection element 1 according to this embodiment will be described.

First, a method of producing a thallium bromide crystal for constituting the crystal part 10 having the metal-containing parts 12 will be described.

A single crystal of thallium bromide is produced by subjecting a commercial thallium bromide raw material of a purity of 99.99% to a purifying treatment, then growing a single crystal ingot by a single crystal growth system, and processing the single crystal ingot.

As the single crystal growth method, for example, the vertical Bridgman method can be used. The single crystal ingot has a diameter of about 3 inches. The single crystal ingot thus grown is sliced by a slicer, followed further by polishing to obtain a 3-inch thallium bromide single crystal wafer having a desired thickness (in this example, a thickness of 1.5 mm).

Next, bromine is selectively removed from both sides of the thallium bromide single crystal wafer produced above, to form the metal-containing parts 12 that contain thallium as metal.

One method for the selective removal may be a method wherein the single crystal wafer produced is disposed in a vacuum heating chamber, the vacuum heating chamber is evacuated by a pump, and the single crystal wafer disposed in the chamber is heated to a temperature of several tens of degrees Celsius to several hundreds of degrees Celsius. By this, bromine present at the surfaces of the single crystal wafer is selectively removed, to form the metal-containing parts 12 in which thallium is contained as metal. This is based on the utilization of the property of bromine such that, while the boiling point of thallium in the atmospheric air is 1,473 degrees Celsius, bromine has an extremely low boiling point of 59 degrees Celsius and has a high vapor pressure.

Another method may be a method wherein a single crystal wafer produced is immersed in a solution such as an etching solution, to elute bromine into the solution and to reduce thallium ions to thallium metal, thereby selectively removing bromine from the wafer surfaces, similarly to the above, and forming the metal-containing parts 12 in which thallium is contained as metal.

Subsequently, after the formation of the metal-containing parts 12 at the surfaces, the single crystal wafer is subjected to vapor deposition of platinum on the metal-containing parts 12 on both sides by such a method as an electron beam vapor deposition method, to form the metal electrodes 13a and 13b.

Next, the single crystal wafer provided on both sides thereof with the metal-containing parts 12 and the metal electrodes 13a and 13b is diced to a desired size (in this example, 5 mm in width and 4 mm in depth) by a dicing device, to obtain the radiation detection element 1.

While preferred examples of the radiation detection element, the radiation detector provided with the same, and the method for producing the radiation detection element have been described above, various modifications and additions can be considered without departing from the gist of the present invention.

For instance, of the metal electrodes 13a and 13b provided on one radiation detection element 1, one side or both sides may be divided by dicing, photolithography or other method, whereby it is possible to obtain a detector having a plurality of detection channels, such as a strip-type detector or a pixel-type detector.

In addition, the timing of the dicing of the radiation detection elements 1 from the single crystal wafer by the dicing device may not necessarily be after the provision of the metal-containing parts and the metal electrodes on both sides of the single crystal wafer, in order that the same effect as above can be obtained.

For example, the radiation detection element may be produced by dicing a single crystal from the polished single crystal wafer by the dicing device, and providing the metal-containing parts and the metal electrodes on both sides of the diced single crystal.

Alternatively, the radiation detection element may be produced by dicing the single crystal wafer provided with the metal-containing parts, by the dicing device, and thereafter providing the diced single crystal with the metal electrodes.

### Nuclear Medicine Diagnosis Device

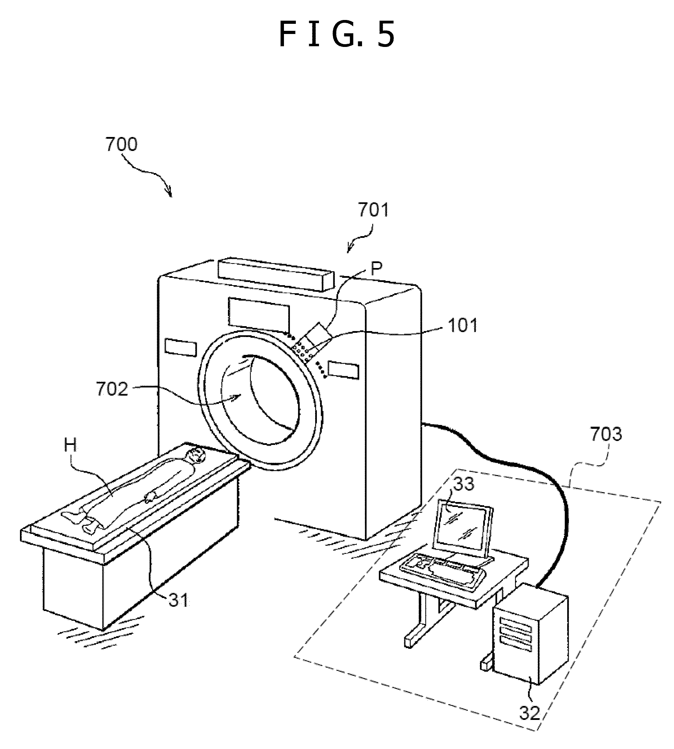

In the next place, the configuration of a nuclear medicine diagnosis device using the radiation detector according to this embodiment will be described, referring to FIGS. 4 and 5.

FIGS. 4 and 5 are each a block diagram of a nuclear medicine diagnosis device using the radiation detector according to one embodiment of the present invention.

First, referring to FIG. 4, a case of applying the radiation detector 101 to a single photon emission computed tomography imaging device (SPECT imaging device) 600 as a nuclear medicine diagnosis device will be described.

In FIG. 4, the SPECT imaging device 600 includes two radiation detection blocks 601A and 601B disposed on upper and lower sides such as to surround a cylindrical measurement region 602 at a central portion, a rotary support base 606, a bed 31, and an image information forming device 603.

Here, the radiation detection block 601A located on the upper side includes a plurality of radiation counting units 611, a unit support member 615, and a light-blocking electromagnetic shield 613. The radiation counting unit 611 includes a plurality of radiation detectors 101, a substrate 612, and a collimator 614. In addition, the radiation detection block 601B located at a lower part also has the same configuration as just-mentioned. Besides, the image information forming device 603 is comprised of a data processor 32 and a display 33.

The radiation detection blocks 601A and 601B are disposed at positions shifted from each other by 180 degrees along the circumferential direction of the rotary support base 606. Specifically, the respective unit support members 615 (only one of them is shown) of the radiation detection blocks 601A and 601B are mounted to the rotary support base 606 at positions spaced from each other by 180 degrees along the circumferential direction. To the unit support member 615, a plurality of radiation counting units 611 including the substrates 612 are attached in a freely detachable manner.

The plurality of radiation detectors 101 are disposed at multiple stages in a region K partitioned by the collimator 614, in the state of being mounted to the substrate 612. The collimator 614 is formed from a radiation-shielding material (for example, lead, tungsten or the like), and forms a multiplicity of radiation passages through which radiation (for example, γ-ray) is propagated.

All the substrates 612 and the collimators 614 are disposed inside the light-blocking electromagnetic shield 613 disposed on the rotary support base 606. The light-blocking electromagnetic shield 613 shields influences of other electromagnetic waves than the γ-ray on the radiation detectors 101 and the like.

In the SPECT imaging device 600 configured in this way, the bed 31 on which a subject H with a radioactive pharmaceuticals administered thereinto is placed is moved in such a manner that the subject H is moved to a position between the pair of radiation detection blocks 601A and 601B. Then, the rotary support base 606 is rotated, whereby the radiation detection blocks 601A and 601B are rotated around the subject H, and detection is started.

Then, when γ-rays are radiated from an accumulation part (for example, an affected part) D in the subject H where the radioactive pharmaceuticals is accumulated, the thus radiated γ-rays pass through the radiation passages of the collimators 614, to be incident on the corresponding radiation detectors 101. Upon this, the radiation detectors 101 output γ-ray detection signals. The γ-ray detection signals are counted on the basis of each γ-ray energy by the data processor 32, and its information and the like are displayed on the display 33.

Note that in FIG. 4, the radiation detection blocks 601A and 601B are rotated as indicated by the thick arrows while being supported by the rotary support base 606, to perform imaging and counting while their angles relative to the subject H are being varied. In addition, the radiation detection blocks 601A and 601B can be moved up and down as indicated by thin arrows, whereby their distances from the subject H can be varied.

As the detector 101 for use in the SPECT imaging device 600 configured as above, there is used the radiation detector that has a circuit configuration as shown in FIG. 3 and has the radiation detection element 1 provided with: the crystal part 10 having the metal-containing parts 12 in which thallium obtained by selectively removing the bromine ion as anion from the thallium bromide crystal and neutralizing the thallium ions is contained as metal; and the metal electrodes 13.

In the next place, referring to FIG. 5, a case of applying the radiation detector 101 of this embodiment to a positron emission tomography (PET) imaging device 700 as a nuclear medicine diagnosis device will be described.

The radiation detector 101 of this embodiment can be used not only for the SPECT imaging device 600 but also for a gamma camera device, a PET imaging device and the like used as a nuclear medicine diagnosis device.

In FIG. 5, a positron emission tomography imaging device (PET imaging device) 700 is configured to include an imaging device 701 having a cylindrical measurement region 702 at a central portion, a bed 31 capable of moving in a longitudinal direction with a subject H supported thereon, and an image information forming device 703. Note that the image information forming device 703 is configured to include a data processor 32 and a display 33.

In the imaging device 701, substrates P each having a multiplicity of the radiation detectors 101 mounted thereon are disposed in such a manner as to surround the measurement region 702.

The PET imaging device 700 configured as above includes an application-specific integrated circuit for digital circuit (digital ASIC; not shown) having a data processing function, and the like, wherein packets having a γ-ray energy value, time, and a detection channel identification (ID) of the radiation detector 101 are formed, and the thus formed packets are inputted to the data processor 32.

At the time of examination, γ-rays radiated from the inside of the subject H due to the radioactive pharmaceuticals are detected by the radiation detectors 101. Specifically, at the moment of annihilation of a positron released from the radioactive pharmaceuticals for PET imaging, a pair of γ-rays is released in opposite directions shifted by 180 degrees from each other. The pairs of γ-rays are detected by different detection channels of the multiplicity of radiation detectors 101. The γ-ray detection signals generated upon the detection are inputted to the relevant digital ASIC, signal processing is conducted as aforementioned, and information on the positions of the detection channels where the γ-ray is detected and information on γ-ray detection time are inputted to the data processor 32.

Then, the data processor 32 counts (coincidence counting) the pair of γ-rays generated upon annihilation of one positron as one, and the positions of the two detection channels where the pair of γ-rays are detected are identified by the data processor on the basis of the position information on the detection channels. In addition, using the count values obtained by the coincidence counting and the position information on the detection channels, the data processor 32 forms tomographic information (image information) of the subject H at an accumulation position of the radioactive pharmaceuticals, or the position of a tumor. The tomographic information is displayed on the display 33.

As the radiation detector 101 for use in the PET imaging device 700 configured as above, there is used the radiation detector that has a circuit configuration as shown in FIG. 3 and has the radiation detection element 1 provided with: the crystal part 10 having the metal-containing parts 12 in which thallium obtained by selectively removing the bromine ion as anion from the thallium bromide crystal and neutralizing the thallium ions is contained as metal; and the metal electrodes 13.

Note that the radiation detector and the nuclear medicine diagnosis device having the same mounted thereon, according to the present invention, are capable of imaging a radioactive pharmaceuticals at a high energy resolution, and can be reduced in size and price; therefore, the radiation detector and the nuclear medicine diagnosis device of the present invention contribute to spreading of these devices and are widely utilized and adopted in this field.

### Description of Reference Symbols

- 1:: Radiation detection element
- 10:: Crystal part
- 11:: Single crystal part
- 12:: Metal-containing part
- 13, 13a, 13b:: Metal electrodes
- 20:: High-voltage power source generation device
- 21:: Resistor
- 22:: Coupling capacitor
- 23:: Charge-sensitive preamplifier
- 24:: Signal processor
- 101:: Radiation detector
- 600:: SPECT imaging device
- 601A, 601B:: Radiation detection blocks
- 602, 702:: Measurement regions
- 603, 703:: Image information forming devices
- 606:: Rotary support base
- 611:: Radiation counting unit
- 612:: Substrate
- 613:: Light-blocking electromagnetic shield
- 614:: Collimator
- 615:: Unit support member
- 700:: PET imaging device
- 701:: Imaging device
- D:: Accumulation part
- H:: Subject
- K:: Region partitioned by collimator
- P:: Substrate

## Claims

1. A radiation detection element comprising:
a crystal part formed from a mixed ionic-electronic conductor that is a compound having a heavy metal ion and an anion bonded to each other;
a first metal electrode formed on one surface of the crystal part; and
a second metal electrode formed on a surface of the crystal part, this surface being opposite to the surface where the first metal electrode is formed,
wherein the crystal part has a metal-containing part on each of the surfaces where the first metal electrode and the second metal electrode are formed, the metal-containing part containing a heavy metal obtained by selectively removing an element of the anion and neutralizing the heavy metal ions.

2. The radiation detection element according to claim 1, wherein the crystal part is formed from the mixed ionic-electronic conductor that is any one of thallium bromide, mercury iodide, lead iodide, bismuth iodide, and indium iodide.

3. The radiation detection element according to claim 1, wherein the first metal electrode and the second metal electrode each include at least one metal of gold, platinum, palladium, and chromium.

4. A radiation detector comprising:
the radiation detection element according to claim 1; and
a signal processor that processes a radiation detection signal outputted from either one of the first metal electrode and the second metal electrode.

5. The radiation detector according to claim 4, further comprising a voltage applying device capable of changing polarity of a voltage to be applied on the radiation detection element.

6. A nuclear medicine diagnosis device comprising:
a substrate fitted with a plurality of radiation detectors, the substrate surrounding a measurement region into which a bed supporting a subject thereon is inserted, and the substrate disposed in surroundings of the measurement region; and
an image information forming device that forms an image by use of information obtained based on radiation detection signals outputted from the plurality of radiation detectors on the substrate,
wherein the radiation detector according to claim 4 is provided as each of the radiation detectors.

7. A method for producing a radiation detection element, the method comprising the steps of:
producing a mixed ionic-electronic conductor that is a compound having a heavy metal ion and an anion bonded to each other;
forming a metal-containing part containing a heavy metal obtained by selectively removing an element of the anion and neutralizing the heavy metal ions, on each of a first surface and a second surface of the crystal formed, the second surface being opposite to the first surface; and
forming a first metal electrode on the first surface where the metal-containing part is formed, and forming a second metal electrode on the second surface where the metal-containing part is formed.

8. The method for producing a radiation detection element according to claim 7,
wherein a crystal formed from a mixed ionic-electronic conductor that is any one of thallium bromide, mercury iodide, lead iodide, bismuth iodide, and indium iodide is produced as the crystal.

9. The method for producing a radiation detection element according to claim 7,
wherein materials of the first metal electrode and the second metal electrode are each at least one metal of gold, platinum, palladium, and chromium.

10. The method for producing a radiation detection element according to claim 7,
wherein the step of forming the metal-containing part is a step of subjecting the crystal to a heat treatment in vacuum.

11. The method for producing a radiation detection element according to claim 7,
wherein the step of forming the metal-containing part is a step of eluting the anion in the crystal into a solution.
